# EUROPÄISCHE PATENTANMELDUNG

(11) **EP 3 205 729 A1**
(43) Veröffentlichungstag der Anmeldung: **16.08.2017**
(21) Anmeldenummer: 16450002.7
(22) Anmeldetag: 11.02.2016
(51) Int. Cl.: C12Q 1/68

(54) **VERFAHREN ZUM NACHWEIS FETALER NUKLEINSÄUREN**

(71) Anmelder: Feichtinger, Wilfried, 2380 Perchtoldsdorf (AT)
(72) Erfinder: Feichtinger, Wilfried, A-2380 Perchtoldsdorf (AT); Feichtinger, Elisabeth, A-2380 Perchtoldsdorf (AT); Palini, Simone, I-47841 Cattolica RN (IT)
(74) Vertreter: Keschmann, Marc

(57) **Zusammenfassung**

Ein Verfahren zum Nachweis fetaler Nukleinsäuren aus dem Überstand des Kulturmediums fetaler Zellkulturen einer in-vitro Fertilisation umfasst die Schritte: a) Entnahme einer Teilmenge des Überstands des in-vitro Fertilisationskulturmediums aus dem zur Kultivierung der befruchteten Eizelle verwendeten Kulturgefäß, wobei mit der Teilmenge von der befruchteten Eizelle verschiedene, intakte zelluläre Strukturen, wie zum Beispiel intakte Trophoblastenzellen oder intakte Blastomeren, entnommen werden, b) Isolierung der in den entnommenen intakten zellulären Strukturen enthaltenen fetalen Nukleinsäuren und c) Durchführung einer Analyse der isolierten fetalen Nukleinsäuren.

## Beschreibung

Die Erfindung betrifft ein Verfahren zum Nachweis fetaler Nukleinsäuren aus dem Überstand des Kulturmediums fetaler Zellkulturen einer in-vitro Fertilisation.

Um die Erfolgsrate bei einer in-vitro Fertilisation (IVF) zu erhöhen, werden die einzelnen Embryonen vor dem Transfer in den Uterus mittels Präimplantationsdiagnostik (PID) morphologisch und molekulargenetisch analysiert. Als Präimplantationsdiagnostik werden zellbiologische und molekulargenetische Untersuchungen bezeichnet, die der Entscheidung dienen, ob ein durch invitro Fertilisation erzeugter Embryo in den Uterus eingepflanzt werden soll oder nicht. Die PID wird hauptsächlich zur Erkennung bestimmter Erbkrankheiten und Besonderheiten der Chromosomen angewendet. Sie kann aber beispielsweise auch zur Auswahl des Geschlechts oder bestimmter erblicher Eigenschaften des Embryos genutzt werden.

Die Präimplantationsdiagnostik (PID) stützt sich derzeit hauptsächlich auf invasive Verfahren wie Blastomeren- oder Trophektoderm-Biopsie, die mit einem kleinen, aber dennoch signifikanten Risiko einer Beschädigung des Embryos einhergehen.

Bei einer Blastomerenbiopsie wird zuerst mit Hilfe von Säure, Laserlicht oder auf mechanischem Weg eine Öffnung in der den Embryo - zu diesem Zeitpunkt (Tag eins bis drei nach der Befruchtung) aus einer Vielzahl von Blastomeren bestehend - umgebenden Zona Pellucida geschaffen.

Anschließend werden dem Embryo mittels einer Saugpipette eine oder zwei Zellen entnommen und im Anschluss genetisch analysiert.

Eine weitere Möglichkeit zur PID ist es den Embryo erst etwa am fünften Entwicklungstag - im sogenannten Blastocystenstadium - zu biopsieren. In diesem Entwicklungsstadium besteht der Embryo aus einer äußeren Zellgruppe, aus der die Plazenta hervorgeht (Trophoblast), und der inneren Zellmasse, aus der sich der Embryo entwickelt (Embryoblast). Bei der sog. Tröphektoderm-Biopsie werden in der Regel mehrere Trophoblasten-Zellen entnommen und im Anschluss genetisch untersucht.

Eine weitere Möglichkeit zur PID ist es die im Blastocoel der Blastocyste enthaltene Flüssigkeit zu entnehmen ("blastocoel puncture") und zu analysieren. Hierbei ist ein Durchdringen der den Embryo umgebenden Zona Pellucida mittels einer Punktionsnadel notwendig, um einen Teil der Blastocoelflüssigkeit aus dem Blastocoel zu entnehmen, was wiederum eine nicht zu vernachlässigende Gefährdung des Embryos bedingt.

Aufgrund dessen ist der Fachmann bestrebt ein Verfahren zur Präimplantationsdiagnostik zu schaffen, welches eine geringst mögliche Gefährdung des Embryos aber dennoch eine hohe Aussagekraft bereitstellt.

Hierzu gibt es gemäß bekanntem Stand der Technik bereits die folgenden Ansätze.

In der WO2013/116889A1 wird beispielsweise ein Verfahren beschrieben, bei dem lediglich der zellfreie Überstand des Kulturmediums fetaler Zellkulturen analysiert wird. Diese Methode ist zwar weitaus weniger invasiv als die vorgenannten Verfahren, als nachteilig ist hierbei jedoch zu sehen, dass keine intakten Zellen analysiert werden, sondern lediglich DNA-Bruchstücke aus abgestorbenen, apoptotischen Zellen innerhalb des Embryos. Im Zuge der Apoptose wird die DNA aus diesen Zellen freigesetzt und tritt durch die Zona Pellucida hindurch in das Kulturmedium über.

Auch in der WO2015/114574A1 wird ein Verfahren zur nichtinvasiven PID eines Embryos beschrieben, wobei ebenfalls eine zellfreie Probe des in-vitro Kulturmediums des Embryos analysiert wird. Auch hier werden lediglich freie Nukleinsäuren - d.h. fragmentierte DNA, RNA, miRNA - welche aufgrund von Apoptose aus der befruchteten Eizelle in das Kulturmedium übergetreten sind, analysiert und keine intakten Zellen.

Nachteilig bei der Analyse von zellfreiem Überstand eines Kulturmediums fetaler Zellkulturen gemäß vorgenanntem Stand der Technik ist es, dass lediglich DNA-Bruchstücke aus abgestorbenen, apoptotischen Zellen, d.h. lediglich freie, fragmentierte Nukleinsäuren, welche aufgrund von Apoptose aus der befruchteten Eizelle in das Kulturmedium übergetreten sind, analysiert werden und keine intakte DNA aus vitalen Zellen. Die Analyse fragmentierter DNA bedingt Probleme bei der Amplifizierbarkeit, Sensitivität und Spezifität der im Zuge der PID vorgenommenen genetischen Analysen, was wiederum die Aussagekraft und Richtigkeit des mittels der Analysen erzielten Ergebnisses verfälschen kann und dadurch die mittels PID angestrebte, gezielte Auswahl eines durch in-vitro Fertilisation erzeugten Embryos negativ beeinflussen kann. Da es keine Goldstandards betreffend die DNA-Extraktion und die Charakterisierung von fragmentierter DNA gibt, besteht zudem die Gefahr, dass die Isolierung und Anreicherung fragmentierter DNA mittels z.B. PCR, biologische Artefakte wie Nullallele, Tri-Allel-Muster, "non-template nucleotide addition" und "Allelic dropout" (Analyse nur eines statt beider Allele) liefert, was wiederum eine falsche Genotypisierung des Embryos bedingen kann.

Die vorliegende Erfindung zielt daher darauf ab, die vorgenannten Nachteile zu vermeiden.

Überraschenderweise wurde festgestellt, dass in Kultur befindliche Blastocysten während ihres Schlüpfvorganges aus der Zona Pellucida - dem sog. "hatching", welches an Tag 5 und 6 der Emryogenese stattfindet, einzelne Zellen (Trophoblastenzellen) in das Kulturmedium abgeben. Weiters wurde beobachtet, dass auch bereits aus der Morula bzw. Blastula (Tag 3 bzw. 4 der Embryogenese) einzelne intakte Blastomeren in das Kulturmedium übertreten.

Zur Lösung der Aufgabe - nämlich ein nicht-invasives, einfach durchzuführendes Verfahren zur PID zu schaffen, welches eine gute Amplifizierbarkeit, eine hohe Sensitivität und Spezifität der genetischen Analysen gewährleistet - sieht die Erfindung daher ein Verfahren der Eingangs genannten Art vor, umfassend die Schritte: a) Entnahme einer Teilmenge des Überstands des in-vitro Fertilisationskulturmediums aus dem zur Kultivierung der befruchteten Eizelle verwendeten Kulturgefäß, wobei mit der Teilmenge von der befruchteten Eizelle verschiedene, intakte zelluläre Strukturen, wie zum Beispiel intakte Trophoblastenzellen oder intakte Blastomeren, entnommen werden, b) Isolierung der in den entnommenen intakten zellulären Strukturen enthaltenen fetalen Nukleinsäuren und c) Durchführung einer Analyse der isolierten fetalen Nukleinsäuren.

Dadurch, dass erfindungsgemäß intakte zelluläre Strukturen aus vitalen Zellen analysiert werden, ist eine exzellente Amplifizierbarkeit, Sensitivität und Spezifität der im Zuge der PID vorgenommenen genetischen Analysen gewährleistet und biologische Artefakte, welche wiederum eine falsche Genotypisierung des Embryos bedingen können, werden weitestgehend vermieden.

Weiters können auch die bei invasiven Verfahren häufig auftretenden Phänomene, wie z.B. Mosaizismus, Mikrochimerismus und Kontaminierung mit Fremd-DNA vermieden bzw. deutlich verringert werden. Aufgrund der vorgenannten Vorteile wird demgemäß auch das Risiko betreffend Fehldiagnosen vermieden. Durch das erfindungsgemäße, nicht-invasive Verfahren wird zudem die Implantationsfähigkeit des Embryos nicht negativ beeinträchtigt und der Embryo wird nicht geschädigt, weil keine zellulären Bestandteile aus der befruchteten Eizelle bzw. keine BlastocoelFlüssigkeit entnommen werden müssen.

Unter dem Begriff "intakte zelluläre Struktur" ist im Rahmen der vorliegenden Erfindung eine intakte, nicht apoptotische Zelle oder eine zumindest den intakten Zellkern einer Zelle umfassende Struktur zu verstehen. Eine vitale Zelle stellt ein strukturell abgegrenztes, eigenständiges und selbsterhaltendes System dar. Eukaryotische Zellen, wie zum Beispiel humane Zellen besitzen einen Zellkern mit einer Kernhülle. Jede vitale Zelle besitzt weiters eine Zellmembran, die die Zelle von der Umgebung abgrenzt. Die wichtigsten sich innerhalb der Zellmembran befindenden Zellstrukturen - neben dem bereits erwähnten Zellkern und der diesen umschließenden Kernmembran - sind Ribososmen, Mitochondrien, endoplasmatisches Retikulum, Golgi-Apparat und Zytoskelett. Die DNA befindet sich bei eukaryotischen Zellen im Zellkern (in Form von Chromosomen organisiert), sowie in den Mitochondrien (mitochondriale DNA). Vitale Zellen enthalten in deren Zellkern die gesamte genetische Information eines Organismus, d.h. intakte, unfragmentierte DNA.

In vorteilhafter Weise ist das erfindungsgemäße Verfahren dahingehend weitergebildet, dass als fetale Nukleinsäure DNA und/oder RNA analysiert wird, was es ermöglicht zur nachfolgenden Analyse standardisierte molekularbiologische Methoden zu verwenden.

Das erfindungsgemäße Verfahren kann insbesondere dahingehend weitergebildet sein, dass vor Schritt a) die Zona Pellucida einer befruchteten Eizelle mechanisch, chemisch oder per Laser geöffnet wird. Hierdurch wird die Wahrscheinlichkeit erhöht, dass Zellen durch die Zona Pellucida aus der befruchteten Eizelle in das Kulturmedium übertreten, da die Zona Pellucida ein natürliches Hindernis für das Austreten von Zellen in das Kulturmedium darstellt.

In vorteilhafter Weise ist das erfindungsgemäße Verfahren dahingehend weitergebildet, dass nach der mechanischen, chemischen oder Laserbehandlung der Zona Pellucida und vor Schritt a) eine weitere mechanische, chemische oder Laserbehandlung an der befruchteten Eizelle vorgenommen wird. In diesem Zusammenhang kann bevorzugt vorgesehen sein, dass die weitere mechanische, chemische oder Laserbehandlung an Tag 3, 4 oder 5 nach der Befruchtung vorgenommen wird. Hierdurch erhält die befruchtete Eizelle einen weiteren Impuls, der wiederum die Wahrscheinlichkeit erhöht, dass Zellen durch die Zona Pellucida aus der befruchteten Eizelle austreten. Dass besagter Schritt an Tag 3, 4 oder 5 nach der Befruchtung vorgenommen wird, liegt darin begründet, dass den Zellen, die man damit zum Übertritt ins Kulturmedium veranlassen will, noch genügend Zeit bleibt dies auch wirklich zu tun, bevor die befruchtete Eizelle spätestens an Tag 6 der Embryogenese in den Uterus der Frau eingesetzt bzw. kryokonserviert werden muss.

Das erfindungsgemäße Verfahren ist bevorzugt dahingehend weitergebildet, dass Schritt a) an Tag 5 oder 6 nach der Befruchtung vorgenommen wird. Da an Tag 5 bzw. 6 nach der Befruchtung - also im sog. Blastocystenstadium - die Wahrscheinlichkeit am höchsten ist intakte zelluläre Strukturen außerhalb der befruchteten Eizelle aufzufinden, weil sich besagte zelluläre Strukturen am ehesten beim sog. Schlüpfvorgang aus der Zona Pellucida, welcher zwischen Tag 5 und 6 der Embryogenese stattfindet, lösen und ins Kulturmedium übertreten, ist dieser Zeitraum zur Probenentnahme am Besten geeignet.

Das erfindungsgemäße Verfahren ist bevorzugt dahingehend weitergebildet, dass die befruchtete Eizelle vor Schritt a) mittels einer Pipette aus dem Kulturmedium entnommen wird und hierbei außerhalb der Zona Pellucida anhaftende Zellen durch gezieltes Absaugen der befruchteten Eizelle im Kulturmedium verbleiben. Durch diesen Vorgang muss nicht die zu analysierende Zelle aus dem Kulturmedium entnommen werden, sondern diese verbleibt im Medium, wenn die befruchtete Eizelle aus selbigem entnommen wird, um bespielsweise kryokonserviert zu werden bis die Testergebnisse vorliegen. Dies erleichtert die Entnahme der intakten zellulären Struktur aus dem Kulturmedium.

Das erfindungsgemäße Verfahren ist bevorzugt dahingehend weitergebildet, dass die Isolierung der in den entnommenen intakten, zellulären Strukturen enthaltenen fetalen Nukleinsäuren (Schritt b) durch Anreicherung, insbesondere einem Verfahren ausgewählt aus einer Gruppe umfassend Zentrifugation, Filtration, Anbindung an Beads, Amplifikation, insbesondere Polymerasekettenreaktion, durchgeführt wird. Durch die Anwendung standardisierter Verfahren zur Ergebnisfindung kann die Fehlerquote so niedrig wie möglich gehalten werden.

Das erfindungsgemäße Verfahren ist bevorzugt dahingehend weitergebildet, dass die Analyse der isolierten fetalen Nukleinsäuren (Schritt c) ein Verfahren ausgewählt aus einer Gruppe umfassend eine Sequenzierung, Amplifikation, in-situ Hybridisierung, insbesondere Fluoreszenz in-situ Hybridisierung, umfasst. Auch hier handelt es sich um standardisierte Verfahren, welche rasch aussagekräftige Ergebnisse liefern.

In vorteilhafter Weise ist das erfindungsgemäße Verfahren dahingehend weitergebildet, dass Erkrankungen, insbesondere monogenetische Erkrankungen, Blutgruppen, Geschlecht, Aneuploidien und HLA-Typen des Embryos detektiert werden. So erhält man bereits vor Beginn der eigentlichen Schwangerschaft - also vor dem Embryonentransfer der durch IVF erhaltenen befruchteten Eizellen in den Uterus der Mutter - Gewissheit über z.B. den Gesundheitsstatus oder das Geschlecht der Embryonen.

Das erfindungsgemäße Verfahren ist bevorzugt dahingehend weitergebildet, dass die Entnahme des Überstands unter dem Mikroskop vorgenommen wird. Die Entnahme des Überstandes unter dem Mikrosop gewährleistet, dass zielgerichtet nur die zur Analyse benötigte intakte zelluläre Struktur aus dem Medium entnommen wird.

Die Erfindung wird nachfolgend anhand eines Ausführungsbeispiels erläutert, ohne auf dieses beschränkt zu sein.

### 1) Embryokultur und Probenentnahme

Menschliche befruchtete Eizellen wurden bis Tag 3 nach der Befruchtung einzeln in 10µl G-1 PLUS Medium (Vitrolife), und von Tag 3 bis Tag 5/6, in 10µl G-2 PLUS Medium (Vitrolife) kultiviert (Inkubation bei 37°C). An Tag 5/6 wurden durch die Zona Pellucida hindurchgetretene Zellen in Hepes-gepuffertem Medium ergänzt mit Protein (5mg Protein/ml) entnommen. Die entnommenen Zellen wurden in ein PCR-Röhrchen enthaltend 5µl TE Puffer überführt und anschließend eingefroren und bei -80°C bis zur Analyse gelagert.

### 2) Detektion und Quantifizierung von genomischer DNA

Die Amplifizierbarkeit der genomischen DNA wurde mittels quantitativer PCR ermittelt. Die Quantifizierung der genomischen DNA wurde unter Verwendung einer Standardkurve erstellt.

### 3) Whole Genome Amplification (WGA)

Die genomische DNA wurde unter Verwendung des PicoPLEX WGA-Kit (Rubicon Genomics, USA) aus 2,5µl des in TE Puffer aufbewahrten Zellsamples gemäß Herstellerverfahren amplifiziert. Die amplifizierte genomische DNA wurde als Matrize in den nachfolgenden PCR-Reaktionen verwendet.

### 4) Weiterführende Analyse

Das amplifizierte Material wurde für Array Comparative Genomic Hybridisation (CGH) an ein genetisches Fachlabor übermittelt.

Alternativ zur teuren DNA-Amplifikation kann ein für die nachfolgenden Analysen ausreichendes Maß an DNA auch mittels der folgenden Methode erreicht werden.
- Isolierung der Zellen aus dem Überstand des Kulturmediums einer fetalen Zellkultur
- Überführung und Kultivierung der Zellen in einer 96 well plate
- Überprüfung unter Mikroskop, ob sich die Zellen teilen
- Isolierung der DNA nach einigen Zellteilungen
- Beweis mittels Powerplex, dass es sich um fetale Zellen handelt.

Diese Methode hat weiters den Vorteil, dass hiermit nachgewiesen werden kann, dass es sich um intakte, vitale Zellen handelt, da sich nur solche in Kultur teilen.

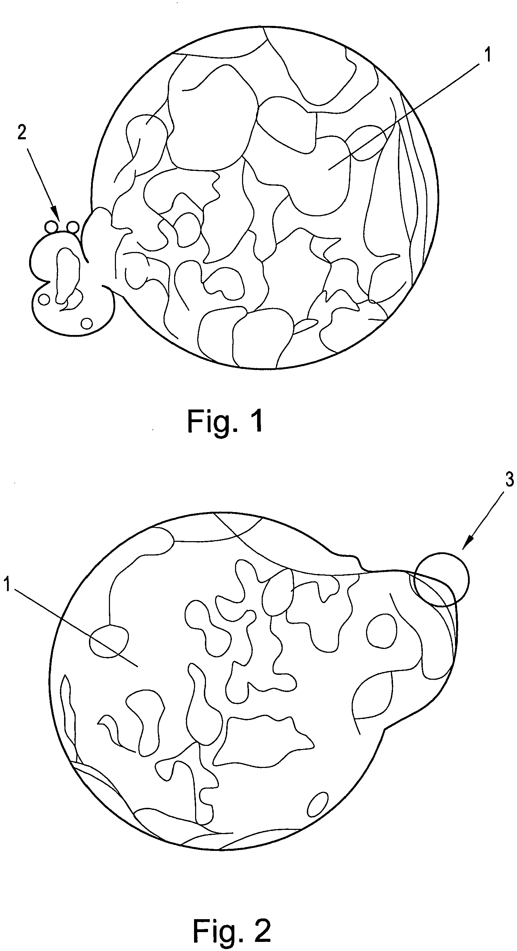

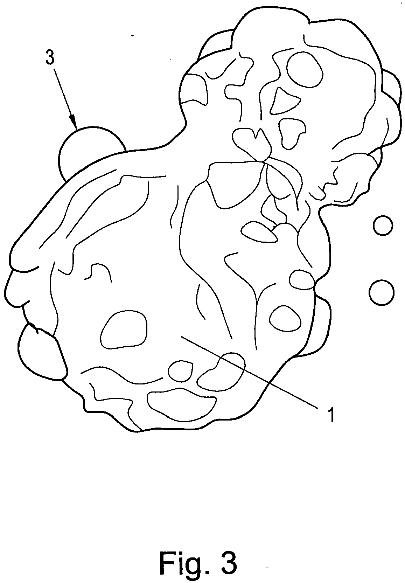

Die Erfindung wird nachfolgend anhand der Fig.1, Fig.2 und Fig.3 näher erläutert, in welchen jeweils befruchtete Eizellen verschiedener Entwicklungsstadien gezeigt sind, aus welchen bereits Trophoblastenzellen bzw. intakte Zellfragmente aus Blastomeren (mit Pfeil markiert) ausgetreten sind.
Fig.1 zeigt eine befruchtete Eizelle 1 im frühen Blastocystenstadium (Tag 4/5 der Embryogenese), samt austretender intakter Blastomere 2.
Fig.2 zeigt eine befruchtete Eizelle 1 im Blastulastadium (Tag 4 der Embryogenese), samt austretender Trophoblastenzelle 3.
Fig.3 zeigt eine befruchtete Eizelle im Blastocystenstadium (Tag 5/6 der Embryogenese), samt austretender Trophoblastenzelle 3.

## Patentansprüche

1. Verfahren zum Nachweis fetaler Nukleinsäuren aus dem Überstand des Kulturmediums fetaler Zellkulturen einer in-vitro Fertilisation umfassend die Schritte: a) Entnahme einer Teilmenge des Überstands des in-vitro Fertilisationskulturmediums aus dem zur Kultivierung der befruchteten Eizelle verwendeten Kulturgefäß, wobei mit der Teilmenge von der befruchteten Eizelle verschiedene, intakte zelluläre Strukturen, wie zum Beispiel intakte Trophoblastenzellen oder intakte Blastomeren, entnommen werden, b) Isolierung der in den entnommenen intakten zellulären Strukturen enthaltenen fetalen Nukleinsäuren und c) Durchführung einer Analyse der isolierten fetalen Nukleinsäuren.

2. Verfahren nach Anspruch 1, **dadurch gekennzeichnet, dass** als fetale Nukleinsäure DNA und/oder RNA analysiert wird.

3. Verfahren nach Anspruch 1 oder 2, **dadurch gekennzeichnet, dass** vor Schritt a) die Zona Pellucida einer befruchteten Eizelle mechanisch, chemisch oder per Laser geöffnet wird.

4. Verfahren nach Anspruch 3, **dadurch gekennzeichnet, dass** nach der mechanischen, chemischen oder Laserbehandlung der Zona Pellucida und vor Schritt a) eine weitere mechanische, chemische oder Laserbehandlung an der befruchteten Eizelle vorgenommen wird.

5. Verfahren nach Anspruch 4, **dadurch gekennzeichnet, dass** die weitere mechanische, chemische oder Laserbehandlung an Tag 3, 4 oder 5 nach der Befruchtung vorgenommen wird.

6. Verfahren nach einem der Ansprüche 1 bis 5, **dadurch gekennzeichnet, dass** Schritt a) an Tag 5 oder 6 nach der Befruchtung vorgenommen wird.

7. Verfahren nach einem der Ansprüche 1 bis 6, **dadurch gekennzeichnet, dass** die befruchtete Eizelle vor Schritt a) mittels einer Pipette aus dem Kulturmedium entnommen wird und hierbei außerhalb der Zona Pellucida anhaftende Zellen durch gezieltes Absaugen der Eizelle im Kulturmedium verbleiben.

8. Verfahren nach einem der Ansprüche 1 bis 7, **dadurch gekennzeichnet, dass** die Isolierung der in den entnommenen intakten, zellulären Strukturen enthaltenen fetalen Nukleinsäuren (Schritt b) durch Anreicherung, insbesondere einem Verfahren ausgewählt aus einer Gruppe umfassend Zentrifugation, Filtration, Anbindung an Beads, Amplifikation, insbesondere Polymerasekettenreaktion, durchgeführt wird.

9. Verfahren nach einem der Ansprüche 1 bis 8, **dadurch gekennzeichnet, dass** die Analyse der isolierten fetalen Nukleinsäuren (Schritt c) ein Verfahren ausgewählt aus einer Gruppe umfassend eine Sequenzierung, Amplifikation, in-situ Hybridisierung, insbesondere Fluoreszenz in-situ Hybridisierung, umfasst.

10. Verfahren nach einem der Ansprüche 1 bis 9, **dadurch gekennzeichnet, dass** Erkrankungen, insbesondere monogenetische Erkrankungen, Blutgruppen, Geschlecht, Aneuploidien und HLA-Typen des Embryos detektiert werden.

11. Verfahren nach einem der Ansprüche 1 bis 10, **dadurch gekennzeichnet, dass** die Entnahme des Überstands unter dem Mikroskop vorgenommen wird.
